# EUROPEAN PATENT SPECIFICATION

(11) **EP 0 344 643 B1**
(45) Date of publication and mention of the grant of the patent: **03.03.1993**
(21) Application number: 89109543.2
(22) Date of filing: 26.05.1989
(51) Int. Cl.: A61K 37/66

(54) **Antiviral pharmaceutical composition**
Virushemmende pharmazeutische Zusammensetzung
Composition pharmaceutique antivirale

(30) Priority: 02.06.1988 GB 8813032
(43) Date of publication of application: 06.12.1989
(73) Proprietor: BOEHRINGER INGELHEIM INTERNATIONAL GmbH, 55218 Ingelheim am Rhein (DE)
(72) Inventor: Suzuki, Nobuo, Prof. Dr., Funabashi Chiba, 273 (JP)

(56) References cited:
- EP-A- 0 295 317

## Description

The present invention relates to the use of dipyridamole for enhancing the antiviral activity of IFN-α, the use of dipyridamole and IFN-α for preparing pharmaceutical preparations for treating such viral infection and to pharmaceutical preparations so produced.

Human interferon α (HuIFN-α) is known to be useful in the treatment of certain forms of tumor, e.g. hairy cell leukemia.

HuIFN-α has also been recognised for some time as a potentially valuable drug for the treatment of some virus infections (see e.g. The Biology of the Interferon System, Elsener/North Holland Biomedical Press 1981, pages 323 to 380). HuIFN-α has been reported for instance as being effective in the treatment of occular Herpes Simplex, when applied at high dosages together with Trifluorothymidin (Sundmacher et al., Lancet II (1978) 687).

The compound 2,6-bis(diethanolamine)-4,8-dipiperidino-pyrimido[5,4-d]pyrimidine, which has the generic name dipyridamole, and its preparations have been described in e.g. US Patent 3,031,450.

Dipyridamole is a well known vasodilator and also has platelet aggregation inhibiting properties; in view of these properties it has found widespread use for many years in the treatment of chronic coronary insufficiency and in the prophylaxis and treatment of heart infarcts as well as in the prophylaxis of arterial thrombosis.

Antiviral activity for dipyridamole has been reported. For instance in East German Patent No. 116.752 (C.A. 85; 37239r) compositions containing dipyridamole for the prophylactic and therapeutic treatment of virus diseases in humans and animals are described. In Acta Virol. (Engl. Ed.), 1977, 21(2): 146-150, it is described that when tested in chick embryo, human diploid and FL cell cultures, dipyridamole inhibited DNA viruses such as vaccinia and pseudorabies virus and those of the Chlamydiacal family and RNA viruses belonging to Piconaviridal, Togaviridal, Orthmyxoviridal and Paramyxoviridal but that Adenoviruses 1 and 3 were resistant to the compound. In a further publication (Acta Virol. [Engl. Ed.] 1978, 22(4): 287-295) various derivatives of dipyridamole were found to be active against a variety of virus such as Coxsakii BI virus, foul plague virus, vaccinia and pseudorabies virus. The publication Z. Allg. Mikrobiol., 1982, 22(9): 661-670, describes work carried out on the in vitro effect of dipyridamole on pseudorabies virus in which it is stated that the antiviral activity of dipyridamole may be due to blocking of the synthesis or of the incorporation of infectious viral DNA into the virus core.

A number of authors have reported the induction of interferon using dipyridamole. Thus in Vopr. Virusol., 32(3): 294-297, 1987, the use of dipyridamole as an agent for preventing acute respiratory viral disease was evaluated. Dipyridamole is described in this article as an interferon inducer. Also in Zh. Mikrobiol. Epidemiol. Immunobiol., (6): 26-30, 1985, is described a report on the "epidemiological effectiveness of dipyridamole, an interferon-inducing agent used for the prevention of influenza and viral acute respiratory diseases." The report claims a pronounced epidermiological effectiveness of dipyridamole.

European Patent application Publication No. 0295317 discloses the enhancement of antitumor effects of human interferons (HuIFN) by dipyridamol.

No authors have reported or suggested the combined uses of dipyridamole and IFN-α in the treatment of or prophylaxis of viral infections, or suggested that any worthwhile results could be expected from such a combined therapy.

Surprisingly the present inventors now have found that dipyridamole itself, whilst having in fact no or negligible antiviral activity, is capable of remarkably enhancing the antiviral activity of IFN-α, especially HuIFN-α.

Broadly speaking the present invention comprises the use of dipyridamole or a pharmaceutically acceptable salt thereof for use in the preparation of a pharmaceutical composition for enhancing the antiviral effects of INF-α.

The resulting pharmaceutical composition can be used in the treatment or prophylaxis of a viral infection of a mammal which treatment or prophylaxis comprises administering to the said mammal dipyridamole or a pharmaceutically acceptable salt thereof in an amount sufficient to enhance the antiviral activity of an IFN-α also administered to the mammal.

Yet a further aspect of the present invention comprises the use of an IFN-α in the manufacture of a pharmaceutical composition for use together with dipyridamole or a pharmaceutically acceptable salt thereof in the treatment or prophylaxis of viral infection of a mammal.

Another aspect of the invention comprises a pharmaceutical composition containing IFN-α and dipyridamole or a pharmaceutically acceptable salt thereof together with conventional pharmaceutical excepients for use in the treatment or prophylaxis of viral infection.

The term IFN-α as used herein includes both human and non-human IFN-α depending on whether therapy or prophylaxis on human or non-human mammals is required.

As human IFN-α (HuIFN-α) all these HuIFN-αs which have antiviral working are to be understood.

The amino acid sequences of various IFN-αs, their preparation by recombinant technology and the preparation of pharmaceutical compositions containing them are described in the literature.

For example the amino acid sequence and recombinant preparation of HuIFN-α₂ₐ is described in European Patent Publication No. 0.043.980; for HuIFN-α_{2b} these are described in European Patent Publication No. 0.032.134; and for HuIFN-α_{2c} they are described in European Patent Publication 0.115.613.

The dipyridamole or salt thereof and interferon should be administered such that they are simultaneously present in the body. Preferably, therefore, they are administered simultaneously or substantially simultaneously.

It is presently considered that the preferred dosages of interferon and dipyridamole for effective antiviral treatment or prophylaxis in humans are those which provide blood levels of at least 0.1 µM, e.g. 0.1 to 5 µM dipyridamole, more preferably 0.75 to 1.5 µM and most preferably about 1 µM and more than 0.01 IU/ml HuIFN-α.

It will be clear that actual dosages may be altered by the attending physician depending upon the circumstances and conditions of the individual patient.

For the use in the practice of the present invention the interferon may be administered by the parenteral route. The dosage and dosage rates are preferably about 2x10³ to 20x10⁶ I.U., e.g. 2x10⁵ to 4x10⁶ I.U. given twice daily in the case of intravenous administration and once daily in the case of intramuscular injection.

The preparation of suitable dosage forms for IFN-α is well known.

To produce a convenient dosage form for parenteral use an appropriate amount of the HuIFN-α may be disolved in 5 % human serum albumin, if necessary containing a suitable buffer solution. This resulting solution is then passed through a bacteriological filter and the filtered solution is distributed between vials under aseptic conditions, each vial containing an appropriate amount of the interferon and, if desired, lyophylised. The glass vials are preferably stored under cool conditions (-20°C) before use. The interferon may be formulated in known manner in order to obtain pharmaceutically usable compositions, the interferon being mixed with a pharmaceutically acceptable carrier substance: conventional carriers and their formulation are described by E.W. Martin in Remingtons Pharmaceutical Sciences to which reference is expressly made.

The dipyridamole or salt thereof can be administered by any of the usual routes of administration, for example orally or parenterally. At present the preferred route of administration is oral. The recommended dosages are 25 to 200 mg, e.g. 75 to 100 mg four times daily for tablets and dragées or 150 to 200 mg twice daily for delayed release forms. By the intravenous route the dipyridamole can be given e.g. by continuous infusion of 3 mg/kg per day.

Dipyridamole is commercially available under the Trade Mark Persantin in a number of forms, for instance injection solution containing 10 mg dipyridamole and dragées containing 25 mg and 75 mg dipyridamole are described in the Rote Liste 1987 published by the Bundesverband der Pharmazeutischen Industrie e.V., D-6000 Frankfurt a.M., West Germany. Suitable dosage forms containing various amounts of dipyridamole can be prepared using standard techniques. In addition a number of special galenic forms have been described in the literature which are aimed at providing either accelerated or delayed (sustained) release and resorption of dipyridamole, for instance the retard capsule form described in European Patent Publication No. 0.032.562; and the instant release form described in European Patent Publication No. 0.068.191. A further delayed release galenic form is described in British Patent No. 2.025.227.

The present invention is predicated on the observation that dipyridamole demonstrated the surprising property of enhancing the antiviral activity of human IFN-α on a virus strain. Thus a test was carried out as described below:

Natural HuIFN-α_{2c} and ¹²⁵I labelled HuIFN-_{2c} preparations; (10⁸ international reference units (I.U.)/mg protein), were obtained from Dr. Karl Thomae GmbH, West Germany. Dipyridamole was provided by Boehringer Ingelheim GmbH (W-Germany). Other chemical agents were purchased from Nakarai Co. Ltd., Japan.

The antiviral action was measured in a yield reduction assay with a strain of the Indiana serotype of vesicular stomatitits virus (VSV) as described in Suzuki et al., Mutation Research, 106: 357-376, 1982. Samples of VSV, produced in human RSa cells with and without pretreatment for 24 hours by dipyridamole and HuIFN-α before virus challenge, were applied to cells of a UV^{r}-1 strain. UV^{r}-1, which is a variant strain of RSa, has an increased resistance to the cell proliferation inhibition effect of interferon but the usual susceptibility of cyto-pathogenic effect (CPE) on VSV (Suzuki et al., J. Gen. Virol., 67: 651-661, 1986). Thus, the effect of HuIFN-α and dipyridamole on virus production could be measured by CPE of VSV or UV^{r}-1 cells in proportion to the production levels without anti-cellular effects being caused by the agents which may remain in the VSV-samples obtained.

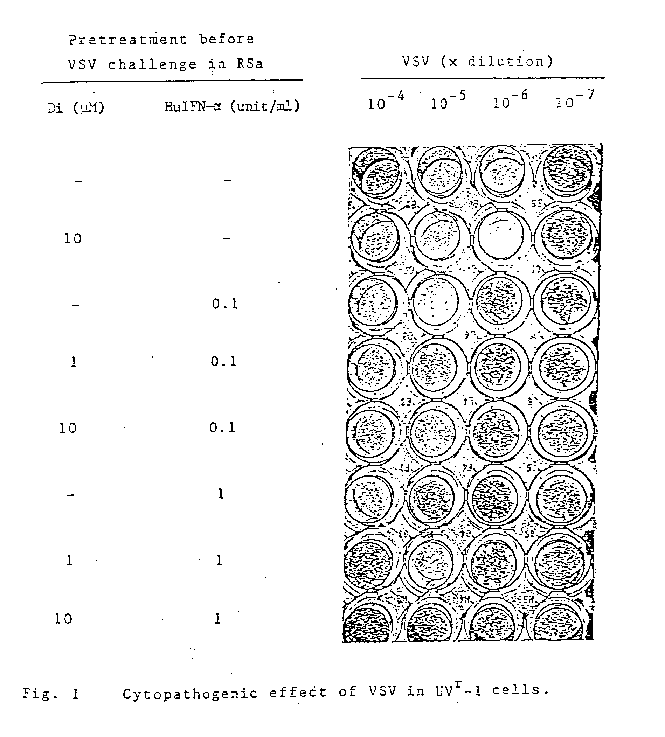

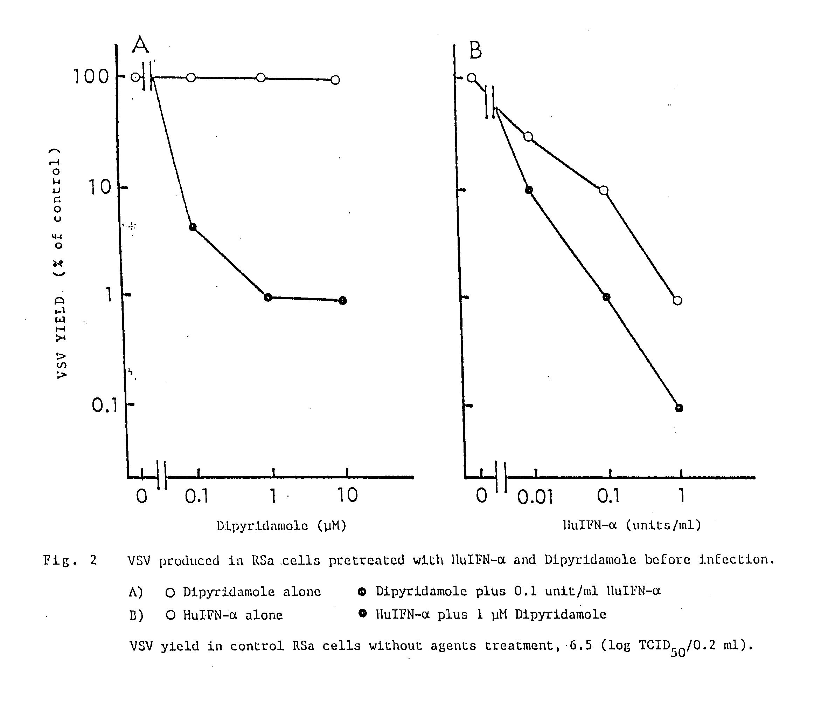

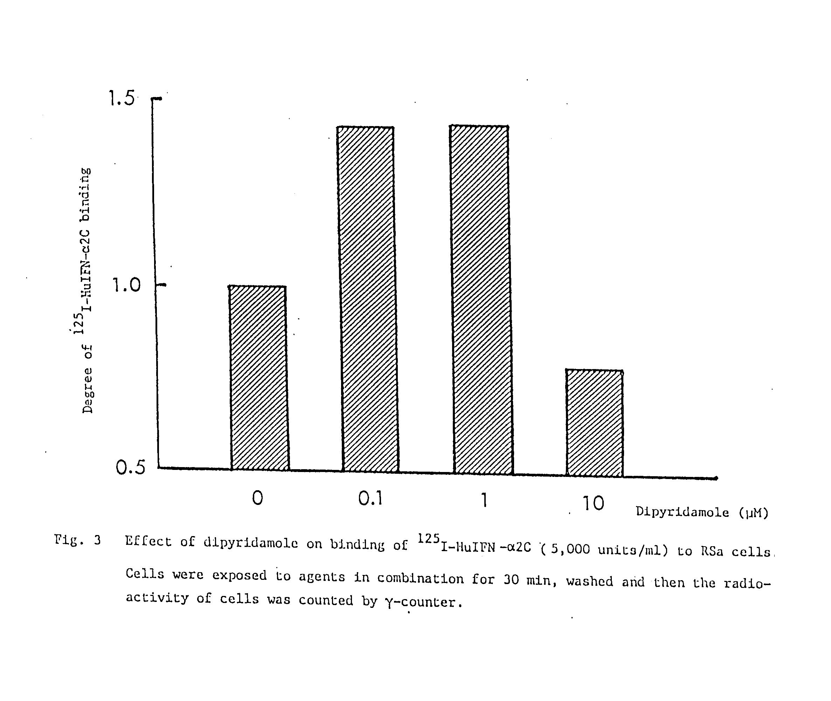

As shown in Fig. 1, CPE was reduced in samples obtained from RSa cells pretreated with dipyridamole and HuIFN-α before VSV infection, although the CPE from pretreatment with dipyridamole alone was much the same as that from non-pretreatment. The appropriate concentration of dipyridamole for the enhancement of HuIFN-α activity was more than 0.1 µM as shown in Fig. 2A. Even if the number of units of HuIFN-α was increased, the enhancement by dipyridamole appeared constant (about ten times the antiviral activity of HuIFN-α alone), as shown in Fig. 2B. The mechanism of the enhancement of antiviral activity of HuIFN-α by dipyridamole has not been elucidated unequivocally yet, but, we have found that dipyridamole enhances HuIFN-α binding capacity. Binding assay was done as described in Suzuki et al., 1986, supra. As shown in Fig. 3, 0.1 µM - 1 µM dipyridamole could enhance the binding capacity in RSa cells to about 1.5 times that in cells non-treated with dipyridamole.

The results presented here are the first which show the surprising effect of dipyridamole on the antiviral action of IFN-α.

### Example 1

### Lyophilized preparation containing 0.04 mg I.U. rHuIFN-α_{2c}

| | |
|---|---|
| (1) rHuIFN-α_{2c} | 0.04 mg (4x10⁶ I.U.) |
| (2) isotonic phosphate buffer pH7 | q.s. |
| (3) human serum albumin | 20.0 mg |
| (4) water for injection | 1.0 ml |

### Preparation

The additives (2) and (3) are dissolved in 2/3 of the total required amount of water. The exact amount of HuIFN-α_{2c} is then added and the solution is brought to the final required volume by adding the remaining water, filtered through 0.22 µM filter and filled into vials, lyophilized and stoppered.

### Examples 2 to 4

Using a process analoguous to Example 1 lyophilized preparations containing the following amounts of rHUIFN-α_{2c} in I.U. were prepared:
2) 2 x 10³
3) 2 x 10⁵
4) 20 x 10⁶

### Example 5

Examples 1 to 4 were repeated using rHUIFN-α₂ₐ and HuIFN-α_{2b}.

### Example 6

### Lyophilized preparation containing 2x10³ - 20x10⁶ I.U. rHuIFN-α₂

| | |
|---|---|
| (1) IFN-α₂ | 2x10³ - 20x10⁶ I.U. |
| (2) KCl | 0.2 mg |
| (3) Na₂PH0₄ · 12H₂0 | 2.9 mg |
| (4) KH₂PO₄ | 0.2 mg |
| (5) NaCl | 8.0 mg |
| (6) human serum albumin | 20.0 mg |
| (7) water for injection | 1.0 ml |

### Preparation

The buffer materials (2), (3), (4), the stabiliser (6), the sodium chloride (5) and the active ingredient (1) were dissolved in the water (7). After sterile filtration the solution was filled into vials under sterile conditions and freeze dried.

### Example 7

Example 6 was repeated using rHuIFN-α₂ₐ and rHuIFN-α_{2b}.

### Example 8

### Dragée containing 75 mg dipyridamole

| Composition | |
|---|---|
| (1) dipyridamole | 7.5 kg |
| (2) lactose | 4.2 kg |
| (3) maize starch | 2.4 kg |
| (4) polyvinylpyrolidine | 0.7 kg |
| (5) magnesium stearate | 0.2 kg |

(1) + (2) + (3) + (4) were mixed and to the mixture was added water to form a moist mass.

The moist mass was passed through a sieve having 1.2 mm spacing and dried at 45 °C in a drying chamber. The dry granules were passed through a sieve having 1 mm spacing and mixed with (5).

The final mixture was pressed to form dragées.

| | |
|---|---|
| Core weight | 150 mg |

The dragée cores so formed were covered by a surface coating in known manner, the coating consisting essentially of sugar and talc. This coating may also contain permitted coloring agents. The finished dragées are polished with wax.

| | |
|---|---|
| Dragée weight | 200 mg |

### Example 9

### Ampoule containing 10 mg dipyridamole

| | |
|---|---|
| dipyridamole | 10 mg |
| tartaric acid | 4 mg |
| polyethylene glycol | 100 mg |
| hydrochloric acid (IN) to adjust to ph 2.7 | q.s. (∼ 0.018 m) |
| water for injection add. | 2 ml |

The dipyridamole and other ingredients were dissolved in water. After sterile filtration the solution is filled into ampoules and sterilized by heating.

### Example 10

An ampoule from Examples 1 to 7 is formulated for injection using water for injection or isotonic saline solution to provide an injection solution of 2 ml.

### Example 11

### Combination preparation containing dipyridamole and HuIFN-α

The contents of the ampoules from Examples 1 to 7 were reconstituted with the contents of the dipyridamole ampoule from Example 9.

The combination preparation of HuIFNα₂ with dipyridamole was stable at room temperature for about 6 hours.

### Example 12

### Capsules containing delayed-release dipyridamole formations

30 kg of rounded tartaric acid starter pellets is sprayed, in a special pan, with a suspension consisting of isopropanole, dipyridamole and polyvinylpyrrolidone until the resulting pellets of active substance contain about 45% dipyridamole.

These pellets are sprayed with a lacquer consisting of methacrylic acid/methyl methacrylate copolymer (brand name Endragit S) and hydroxypropylmethylcellulose phthalate (brand name HP 55) in a weight ratio 85:15 to 50:50.

The organic lacquer solution also contains plasticiser and talc. Two pellet components are sprayed with 5% and 7% of coating and different ratios of lacquer components within the limits specified and are mixed together.

In a special capsule making machine, the quantity of pellets corresponding to 150 - 200 mg of dipyridamole are packed into an appropriate sized capsules.

## Claims

1. The use of dipyridamole or a pharmaceutically acceptable salt thereof in the manufacture of a pharmaceutical composition suitable for enhancing the antiviral effect of an IFN-α when administered to a mammal requiring antiviral treatment and to whom said IFN-α is also administered.

2. The use of IFN-α in the manufacture of a pharmaceutical composition for use together with dipyridamole or a pharmaceutically acceptable salt thereof in the treatment or prophylaxis of viral infection of a mammal.

3. A pharmaceutical composition containing IFN-α and dipyridamole or a pharmaceutically acceptable salt thereof for use in the treatment or prophylaxis of a viral infection in a mammal.

## Patentansprüche

1. Verwendung von Dipyridamol oder eines pharmazeutisch annehmbaren Salzes davon zur Herstellung einer pharmazeutischen Zubereitung, welche geeignet ist, die Antivirus-Wirkung von IFN-alpha zu verstärken, wenn sie einem Säugetier, welches einer Antivirus-Behandlung bedarf und dem ausserdem das genannte IFN-alpha verabreicht wird.

2. Verwendung von IFN-alpha zur Herstellung einer pharmazeutischen Zubereitung für die Verwendung zusammen mit Dipyridamol oder einem pharmazeutisch annehmbaren Salz davon für die Therapie oder Prophylaxe von Virus-Infektionen bei einem Säugetier.

3. Pharmazeutische Zubereitung, enthaltend IFN-alpha und Dipyridamol oder ein pharmazeutisch annehmbares Salz davon für die Therapie oder Prophylaxe von Virus-Infektionen bei einem Säugetier.

## Revendications

1. Utilisation du dipyridamole ou d'un sel pharmaceutiquement acceptable de celui-ci pour la préparation d'une composition pharmaceutique capable d'augmenter l'effet antiviral d'un IFN α lorsqu'elle est administrée à un mammifère qui nécessite un traitement antiviral et auquel ledit IFN α est également administré.

2. Utilisation d'un IFN α pour la préparation d'une composition pharmaceutique utilisable conjointement avec le dipyridamole ou un sel pharmaceutiquement acceptable de celui-ci dans le traitement ou la prophylaxie d'une infection virale chez un mammifère.

3. Composition pharmaceutique contenant un IFN α et du dipyridamole ou un sel pharmaceutiquement acceptable de celui-ci utilisable dans le traitement ou la prophylaxie d'une infection virale chez un mammifère.
